# EUROPEAN PATENT APPLICATION

(11) **EP 2 286 680 A1**
(43) Date of publication of application: **23.02.2011**
(21) Application number: 09758197.9
(22) Date of filing: 12.05.2009
(51) Int. Cl.: A23L 3/24, A23B 7/00, A61L 2/06

(54) **PASTEURIZATION METHOD AND PASTEURIZATION APPARATUS**

(30) Priority: 06.06.2008 JP 2008149527
(71) Applicant: Liquid Gas Co., Ltd., Osaka 541-0048 (JP); Kyushu University, National University Corporation, Higashi-ku Fukuoka-shi Fukuoka 812-8581 (JP)
(72) Inventor: FUJIWARA Hiroshi, Osaka-shi Osaka 541-0048 (JP); HIGO Keizo, Osaka-shi Osaka 541-0048 (JP); SHIMODA Mitsuya, Fukuoka-shi Fukuoka 812-8581 (JP)
(74) Representative: Grosse, Felix Christopher
(86) International application number: PCT/JP2009/058836
(87) International publication number: WO 2009/147930

(57) **Abstract**

In order to provide a pasteurizing method that provides high pasteurizing effect in pasteurization of an object to be treated and that hardly causes denaturation of the treated object, the method includes a dropping step of dropping the object in a steam zone and a contacting step of causing the dropping object to come into contact with the steam.

## Description

### Technical Field

The present invention relates to a pasteurizing method and a pasteurizing apparatus for pasteurizing an object to be treated by heating the object with steam.

### Background Art

As a prior art, as disclosed in Fig. 1 of Patent Document 1 for instance, there are known a method and an apparatus for pasteurizing objects to be treated(fresh vegetables such as cut vegetables) by causing the objects to contact superheated steam while continuously conveying these objects on a conveyer.

### Prior Art Document

### Patent Document

Patent Document 1: Japanese Patent Application "Kokai" No. 2004-33202

### Summary of the Invention

However, in the case of pasteurizing treatment by causing an object to contact with steam while conveying it on a conveyer, it is difficult for the entire surface of the treated object to contact the stream uniformly in a short time. Hence, it is necessary to extend the contacting period, which can cause deterioration in the freshness of the treated object (vegetable such as cut vegetable). Further, in case the steam is superheated steam, it is difficult for the steam to generate condensation latent heat instantly. For this reason, it is needed to extend the contacting period and such extension of contacting period causes such troubles as unneeded conduction of heat to the inner part of the treated object (in particular its surface portion) and denaturation due to drying (for example, in case the treated object is a fresh food product, its inside may be denatured or drying may cause reduction in its freshness).

The present invention has been made in view of the above-described state of the art. Its object is to provide a pasteurizing method and a pasteurizing apparatus that provide high pasteurizing effect at the time of heating pasteurization of the treated object and that can hardly cause denaturation of the treated object.

For accomplishing the above-noted object, according to an inventive pasteurizing method described in the first characterizing feature of the present invention, a method of pasteurizing an object to be treated by heating the object with steam, the method comprises the steps of dropping the object into a steam zone and causing the dropping object to contact the steam.

### [Function and Effect]

According to the first characterizing feature of the invention, in the method of pasteurizing an object to be treated by heating the object with steam, in the steam zone, the steam contacts the treated object for its entire surface, so the surface of the treated object can be heated in a uniform manner, whereby high pasteurizing effect is enhanced. Moreover, as the contacting period of the contact between the treated object and the steam is very short, the denaturation of the object due to heating hardly occurs.

According to an inventive pasteurizing method described in the second characterizing feature of the present invention, said steam is saturated steam.

### [Function and Effect]

According to the second characterizing feature of the present invention, as saturated steam has large heat content and condenses upon its contact with the surface of the treated object to be changed into water, a large condensation latent heat can be generated. In particular, in case the partial pressure of noncondensable gas such as air is low, the generation rate (heat transfer speed) of the condensation latent heat is increased extremely.
Therefore, the surface of the treated object is heated within a very short time, so that the heat hardly conducts to the inner part of the object. As a result, the denaturation of the treated object due to heating can be prevented even more effectively.

According to the inventive pasteurization method described in the third characterizing feature of the present invention, said treated object is a fresh food product.

### [Function and Effect]

According to the third characterizing feature of the present invention, it is possible to pasteurize by heating the entire surface of the fresh food product while preventing deterioration in the freshness of the fresh food product.

According to the inventive pasteurization method described in the fourth characterizing feature of the present invention, a substance providing a pasteurizing effect or an effect of restricting propagation of bacteria is added to the steam.

### [Function and Effect]

According to the fourth characterizing feature of the present invention, the pasteurizing effect for the treated object is further enhanced.

According to the inventive pasteurizing apparatus described in the fifth characterizing feature of the present invention, there is provided an apparatus for pasteurizing an object to be treated by heating the object with steam, the apparatus comprising:
a treated object charging portion provided at an upper end of the apparatus;
a treated object discharging portion provided at a lower end of the apparatus;
said treated object charging portion and said treated object discharging portion being communicated to each other;
an enclosure member defining therein a dropping passageway for causing the treated object to drop from said charging portion to said discharging portion;
a first conveying means for conveying the treated object to said charging portion;
a steam supplying unit for supplying steam to the dropping passageway of the enclosure member;
a steam rise restricting mechanism for restricting rising of steam supplied to the dropping passageway; and
a second conveying means for receiving the object dropped in the dropping passageway and conveying the object to the outside of the enclosure member;
wherein as said object is conveyed to the charging portion by the first conveying means and dropped, the object comes into contact with the steam supplied from the seam supplying unit in the dropping passageway, thus being pasteurized by heating.

### [Function and Effect]

According to the fifth characterizing feature of the present invention, as the object is conveyed to the charging portion by the first conveying means and dropped, the object comes into contact with the steam supplied from the seam supplying unit in the dropping passageway, thus being pasteurized by heating. Therefore, the period of contact between the object and the steam is very short, so that denaturation of the treated object due to heating hardly occurs.
Also, although the steam supplied from the steam supplying unit may tend to rise in the dropping passageway of the enclosure member to be discharged from the discharging portion, the steam rise restricting mechanism restricts such rising of steam. Therefore, the steam can easily stay within the dropping passageway of the enclosure member. As a result, the object as being entirely covered with the steam can easily drop in the dropping passageway, so that the surface of the treated object can be heated effectively and uniformly Hence, there is achieved high pasteurizing effect.

According to the inventive pasteurizing apparatus described in the sixth characterizing feature of the present invention, said steam rise restricting mechanism comprises an opening/closing lid provided at said charging portion, with the lid being openable/closable.

### [Function and Effect]

According to the sixth characterizing feature of the present invention, with the opening/closing lid provided at the charging portion, the steam present inside the dropping passageway can hardly escape upwards, so the pasteurizing effect of the treated object through its contact with steam can be improved with a simple construction.

According to the inventive pasteurizing apparatus described in the seventh characterizing feature of the present invention, said steam rise restricting mechanism comprises an air curtain provided at the charging portion.

### [Function and Effect]

According to the seventh characterizing feature of the present invention, with supplying of air alone, upward escaping of the steam inside the dropping passageway can be restricted without providing any mechanical device and the pasteurizing effect of the treated object through its contact with steam can be improved.

According to the inventive pasteurizing apparatus described in the eighth characterizing feature of the present invention, there is provided a cooling unit for cooling the object that has contacted the steam.

### [Function and Effect]

According to the eighth characterizing feature of the present invention, as the treated object which has been pasteurized by heating through its contact with steam is cooled by the cooling unit, heat conduction to the inner part of the pasteurized object due to water droplets condensed on the surface of the object is prevented. Hence, denaturation of the treated object due to heating can be prevented in even more reliable manner.

According to the inventive pasteurizing apparatus described in the ninth characterizing feature of the present invention, said steam supplying unit includes an adding means for adding to the steam a substance having a pasteurizing effect or an effect of restricting bacteria propagation.

### [Function and Effect]

According to the ninth characterizing feature of the present invention, with provision of the adding means, the pasteurizing effect can be further enhanced.

### Brief Description of the Drawings

[Fig. 1] is a diagram showing the general construction of a pasteurizing apparatus according to the first embodiment of the present invention,
[Fig. 2] is a diagram showing the general construction of a pasteurizing apparatus according to the second embodiment of the present invention,
[Fig. 3] is a diagram showing the general construction of a pasteurizing apparatus according to the third embodiment of the present invention,
[Fig. 4] is a diagram showing the general construction of a pasteurizing apparatus according to the fourth embodiment of the present invention,
[Fig. 5] (a), (b) are diagrams showing the general construction of a pasteurizing apparatus according to the fifth embodiment of the present invention, and
[Fig. 6] is a diagram showing the general construction of a pasteurizing apparatus according to the sixth embodiment of the present invention,

### Modes of Embodying the Invention

### [First Embodiment]

### (Pasteurizing Method)

Next, an embodiment relating to a pasteurizing method of the present invention will be described.
This embodiment includes (1) a dropping step and (2) a contacting step to be described later. When necessary, the method can further include e.g. (3) a cooling step.
(1) Dropping Step
   An object to be treated (e.g. a fresh food product of vegetable, fruit, etc.) is dropped from a predetermined height.
(2) Contacting Step
   Next, into a dropping passageway for the treated object, saturated steam at from about 95°C to 100°C is supplied to cause the saturated steam to contact the object being dropped in its saturated steam zone from the entire periphery of the object, thus pasteurizing the object by heating.
(3) Cooling Step
   Cooling water is jetted against the treated object which has been pasteurized by heating through its contact with the saturated steam, thereby to cool this object.
The pasteurizing method of this embodiment is a method of pasteurizing an object to be treated while the object is being dropped and caused to contact saturated steam. As the dropping object comes into contact with the steam over the entire surface of the object, the surface of the treated object can be effectively and uniformly heated, so high pasteurizing effect can be achieved. Moreover, the period of contact between the treated object and the saturated steam is very short, so denaturation of the treated object due to heating hardly occurs.
Incidentally, saturated steam has high heat content and is converted into water upon its condensation resulting from its contact with the surface of the object. Hence, large condensation latent heat can be generated. In particular, in case the partial pressure of noncondensable gas such as air is low, the generation rate (heat transfer speed) of the condensation latent heat is increased extremely.
Therefore, the surface of the treated object is heated within a very short time, so that the heat is hardly conducted to the inner part of the object. As a result, the denaturation of the treated object due to heating can be prevented even more effectively.

### (Construction of Pasteurizing Apparatus 1)

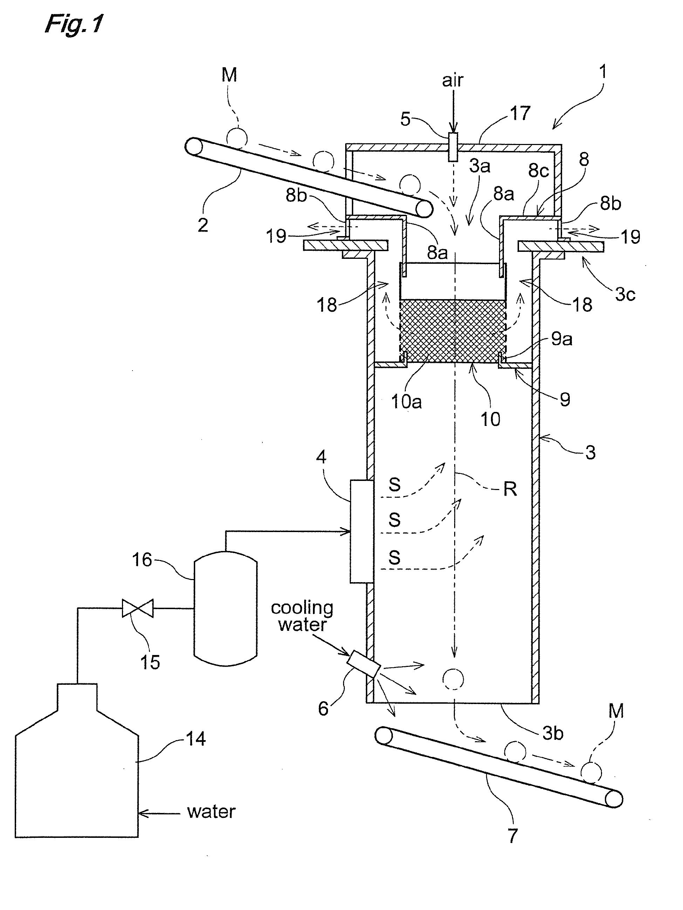

Next, with reference to Fig. 1, there will be described an embodiment of a pasteurizing apparatus 1 capable of implementing the above-described pasteurizing method.
Fig. 1 is a vertical section schematically showing the construction of the pasteurizing apparatus 1 (First Embodiment) relating to the present invention.
As shown in Fig. 1, the pasteurizing apparatus 1 of this embodiment includes a first conveyer 2 (an example of a "first conveying means"), an enclosure member 3, a steam supplying nozzle 4 (an example of a "steam supplying unit), an air supplying nozzle 5 (an example of a "steam rise restricting mechanism"), a cooling water supplying nozzle 6 (an example of a "cooling unit") and a second conveyer 7 (an example of a "second conveying means").

The first conveyer 2 is disposed upwardly of a casing 8 provided in a flange portion 3c of the enclosure member 3. In operation, the first conveyer 2 can convey objects to be treated M (e.g. fresh food products such as vegetables, fruits) to above the casing 8 and charge these objects one after another continuously to the inner cylindrical portion 8a of this casing 8.

The enclosure member 3 is a cylindrical hollow tube and includes an upper opening portion 3a and a lower opening portion 3b at its upper end and lower end, respectively. The upper opening portion 3a and the lower opening portion 3b are communicated to each other and the enclosure member 3 defines therein a dropping passageway R for dropping the objects M from the upper opening portion 3a to the lower opening portion 3b.

At an upper portion of the inner wall of the enclosure member 3, there is provided a supporting portion 9 for supporting a hollow cylindrical tube 10. The supporting portion 9 includes, at its leading end, a raised piece 9a raised along the axial direction of the enclosure member 3. When the cylindrical tube 10 is placed on the supporting portion 9, the raised piece 9a is located on the inner side of the cylindrical tube 10, thus fixing the cylindrical tube in position in the horizontal direction.

The cylindrical tube 10 has a smaller outer diameter than the inner diameter of the enclosure member 3 and the lateral wall of this tube 10 includes a mesh-like vent hole 10a for establishing communication between the inner side and the outer side of the cylindrical tube 10.

At the peripheral edge of the upper opening portion 3a of the enclosure member 3, there is formed a flange portion 3c. On this flange portion 3c, there is disposed a casing 8 having a cylindrical shape. The casing 8 includes an inner cylindrical portion 8a (the outer diameter of the inner cylindrical portion 8a is smaller than the inner diameter of the enclosure member 3 and the top and bottom of the inner cylindrical portion 8a are open) and a support connecting portion 8b which respectively extend continuously from a ceiling portion 8c. The lower end edge of the support connecting portion 8b is supported by the flange portion 3c. The inner cylindrical portion 8a is disposed within the upper opening portion 8a and the lower end of the inner cylindrical portion 8a is fitted inside the cylindrical tube 10 disposed on the supporting portion 9. Meanwhile, the inner cylindrical portion 8a of the casing 8 and the cylindrical tube 10 are communicated to the dropping passageway R of the enclosure member 3, thus forming some parts of this dropping passageway R.

The air supplying nozzle 5 is disposed upwardly of the casing 8 and is supported to a frame 17 mounted on the ceiling portion 8c of the casing 8. In operation, an amount of air supplied by an air compressor (not shown) is injected through the air supplying nozzle 5 to the inner cylindrical portion 8a of the casing 8 so as to restrict rising displacement of saturated steam supplied to the dropping passageway R.

The steam supplying nozzle 4 is mounted to the lateral face of the lower portion of the enclosure member 3.
In operation, superheated steam generated by a boiler 14 is first adjusted in its pressure by a depressurizing valve 15 and then introduced into a saturated steam generating unit 16. Then, as the superheated steam comes into contact with water stored in this saturated steam generating unit 16, there is generated saturated steam at about 100°C. The saturated steam S supplied from the saturated steam generating unit 16 is injected through the steam supplying nozzle 4 into the dropping passageway R of the enclosure member 3.

The cooling water supplying nozzle 6 is mounted to the lateral face of the enclosure member 3 at a position lower than the steam supplying nozzle 4. In operation, an amount of cooling water supplied from a cooling water supplying unit (not shown) is injected through the cooling water supplying nozzle 6 and jetted against or sprayed over the treated object M which has contacted the saturated steam, thus cooling this object M.

The second conveyer 7 is disposed downwardly of the enclosure member 3 and is configured to receive the object M dropped through the lower opening portion 3b of the enclosure member 3 and conveying this object M to the outside of the enclosure member 3.

### [Pasteurizing Treatment]

Next, there will be explained an example of a method of pasteurizing a large amount or number of objects M continuously with using the pasteurizing apparatus 1 having the above-described construction.
First, a worker places the objects M to be pasteurized (e.g. fresh food products of vegetables, fruits, etc.) one after another onto the first conveyer 2. Then, these objects M placed on the first conveyer 2 are conveyed to the position upwardly of the casing 8 and then charged into the inner cylindrical portion 8a of the casing 8 continuously.

The objects M charged into the inner cylindrical portion 8 are caused to drop by gravity in the dropping passageway R of the enclosure member 3, in the course of which the objects come into contact with the saturated steam being supplied from the steam supplying nozzle 4, thus being pasteurized by heating. That is, with the pasteurizing apparatus 1 according to the present embodiment, as the treated object M comes into contact with saturated steam in the course of its dropping, the period of contact between this object M and the saturated steam is limited very short, so denaturation of the treated object M due to heating hardly occurs.

Also, since the saturated steam supplied form the steam supplying nozzle 4 has less density than air, this steam rises in the dropping passageway R of the enclosure member 3, so that the steam may escape through the cylindrical tube 10 and the inner cylindrical portion 8a of the casing 8 to the outside of the enclosure member 3. However, with the pasteurizing apparatus 1 according to the instant embodiment, the apparatus is configured such that such rising of the saturated steam is effectively restricted by the pressure of air supplied from the air supplying nozzle 5. Hence, the saturated steam can be kept within the dropping passageway R of the enclosure member 3. As a result, there can be readily realized the condition of the treated object M being dropped while being entirely entrapped in steam. So, the surface of the treated object M can be heated in an effective manner; hence, the pasteurizing effect is high.

Incidentally, with the pasteurizing apparatus 1 according to the instant embodiment, the inner wall of the upper portion of the enclosure member 3, the inner cylindrical portion 8a and the ceiling portion 8c of the casing 8, etc. together form a discharge passageway 18 for the saturated steam. And, the air vent hole 10a of the cylindrical tube 10 and the discharging portion 19 provided adjacent the support connecting portion 8b of the casing 8 are communicated to each other via the discharge passageway 18. That is, a portion of the saturated steam which has risen in the dropping passageway R of the enclosure member 3 into the cylindrical tube 10 passes the vent hole 10a of the cylindrical tube 10 and passes the discharge passageway 18 and then is discharged through the discharging portion 19 to the outside of the enclosure member 3.

The objects M heat-pasteurized through their contact with the saturated steam are cooled through their contact with cooling water in the form of mist sprayed from the cooling water supplying nozzle 6. Through this cooling step, heat conduction to the inner part of the pasteurized treated products due to the water droplets condensed on the surfaces of the objects M can be prevented. Hence, denaturation of the treated objects M due to heating can be prevented even more reliably.

The resultant cooled treated objects M are caused to drop through the lower opening portion 3b of the enclosure member 3 to be conveyed continuously to the outside of the enclosure member 3. Hence, a great amount or number of treated objects M can be heat-pasteurized efficiently in a short period of time.

### [Second Embodiment]

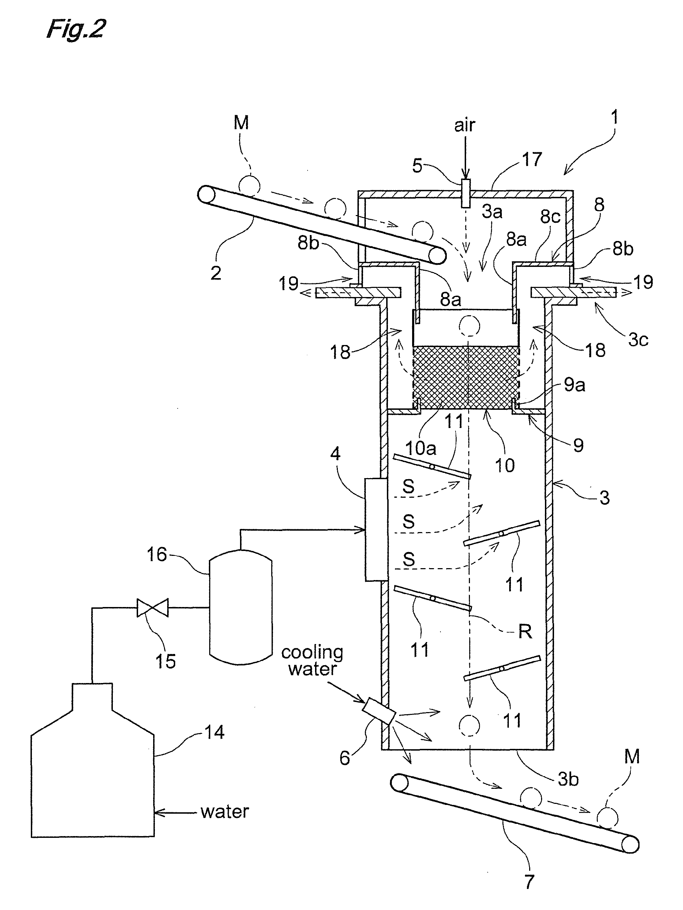

Next, with reference to Fig. 2, there will be described a second embodiment of the pasteurizing apparatus 1 relating to the present invention. In this, in order to avoid redundancy in explanation, only those arrangements, functions and effects different from those of the First Embodiment will be explained, and regarding the same members or components as those in the first embodiment, they will be given the same reference numerals and explanation thereof will be omitted.

Fig. 2 is a vertical section schematically showing the construction of the pasteurizing apparatus 1 (Second Embodiment) relating to the present invention.
As shown in Fig. 2, in the pasteurizing apparatus 1 according to the instant embodiment, inside the enclosure member 3, there are provided a plurality of baffle plates 11 in a staggered layout in vertically spaced relationship with each other. Each baffle plate 11 is inclined such that one terminal end thereof on the side of the dropping passageway R is located more downward than the other terminal end thereof and at least the terminal end on the side the dropping passageway R extends to intersect this dropping passageway R.

In this embodiment, when the treated object M is dropped in the dropping passageway R of the enclosure member 3, the object M is dropped while hitting the respective baffle plates 11. As a result, it takes a longer time for the object M to drop from the upper opening portion 3a to the lower opening portion 3b (this period will be referred to as the "dropping period" hereinafter) than the free fall arrangement in the First Embodiment.
As a result, the period of contact between the treated object M and the saturated steam is longer (that is, a longer period of pasteurizing treatment with the saturated steam), so that the object M can be pasteurized even more reliable manner.

That is, according to this embodiment, through appropriate adjustment of the inclination angles of the baffle plates 11 and/or the number of these plates 11, it is possible to freely adjust the period of pasteurization treatment for the object M. Therefore, it becomes easily possible to set appropriate pasteurizing treatment conditions, depending on the kind of the treated object M. Hence, the heat-pasteurizing treatment can be carried out even more efficiently.

### [Third Embodiment]

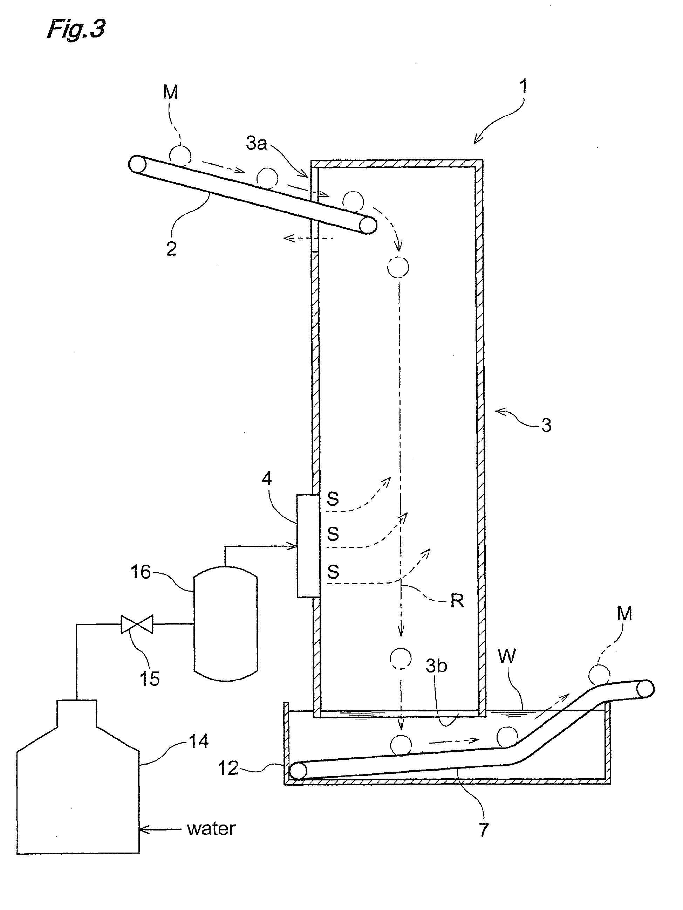

Next, with reference to Fig. 3, there will be described the Third Embodiment of the pasteurizing apparatus 1 relating to the present invention. In this, in order to avoid redundancy in explanation, only those arrangements, functions and effects different from those of the First Embodiment will be explained, and regarding the same members or components as those in the first embodiment, they will be given the same reference numerals and explanation thereof will be omitted.

Fig. 3 is a vertical section schematically showing the construction of the pasteurizing apparatus 1 (Third Embodiment) relating to the present invention.
As shown in Fig. 3, in the pasteurizing apparatus 1 according to the instant embodiment, the upper opening portion 3a is formed in the upper lateral face of the enclosure member 3 and the first conveyer 2 is inserted into this upper opening portion 3a. Also, downwardly of the enclosure member 3, there is provided a water reservoir tank 12 for sealing the lower opening portion 3b of the enclosure member 3 with water.
In operation, the treated objects M are conveyed by the first conveyer 2 to the upper opening portion 3a and caused to drop in the dropping passageway R of the enclosure member 3, in the course of which the objects come into contact with saturated steam supplied from the steam supplying nozzle 4 to be heat-pasteurized thereby. Then, these heat-pasteurized objects M are dropped into the water W held in the water reservoir tank 2 to be cooled thereby. Thereafter, these cooled objects M are conveyed on the second conveyer 7 from the inside of the water reservoir tank 12 to the outside of this water reservoir tank 12 and to the outside of the enclosure member 3 in continuous manner.

In this embodiment, the lower opening portion 3b of the enclosure member 2 is sealed with water. This arrangement makes it difficult for the saturated steam supplied from the steam supplying nozzle 4 to escape through the upper opening portion 3a. As a result of this arrangement, the air supplying nozzle 5 ("steam rise restricting mechanism") provided in the First Embodiment and in the Second Embodiment described above becomes unnecessary.
Further, the object M heat-pasteurized through the contact with saturated steam is cooled as being dropped into water W held in the water reservoir tank 12. With this arrangement, the cooling water supplying nozzle 6 ("cooling unit") provided in the First Embodiment and in the Second Embodiment described above becomes unnecessary also.

That is, the water reservoir tank 12 provided in this embodiment provides the two functions of the steam rise restricting mechanism and of the cooling unit provided in the First Embodiment and Second Embodiment described above. Hence, the construction of the pasteurizing apparatus 1 can be even more simplified.

### [Fourth Embodiment]

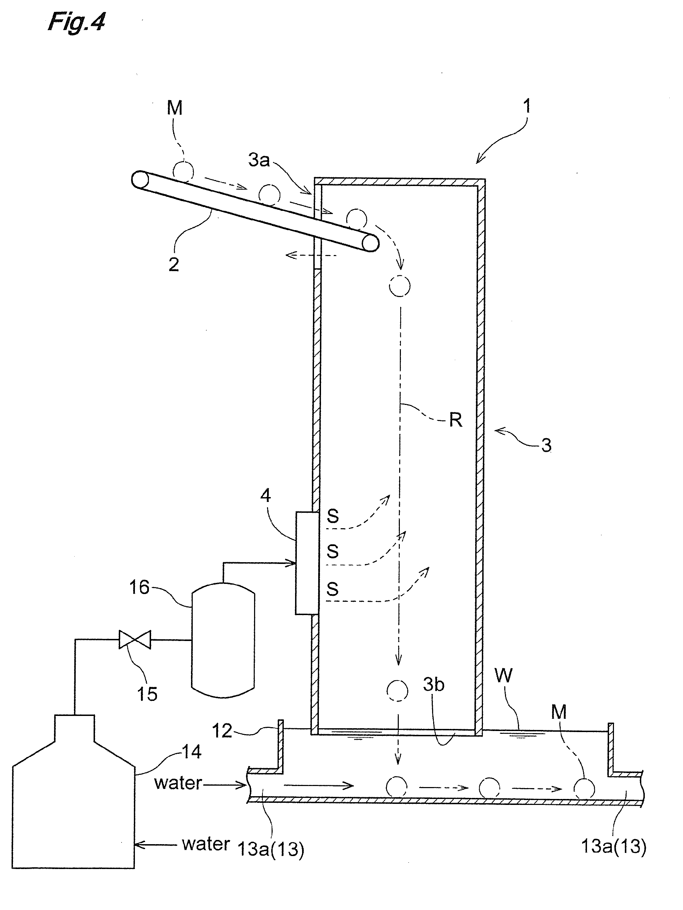

Next, with reference to Fig. 4, there will be described the Fourth Embodiment of the pasteurizing apparatus 1 relating to the present invention. In this, in order to avoid redundancy in explanation, only those arrangements, functions and effects different from those of the Third Embodiment will be explained, and regarding the same members or components as those in the first embodiment, they will be given the same reference numerals and explanation thereof will be omitted.

Fig. 4 is a vertical section schematically showing the construction of the pasteurizing apparatus 1 (Fourth Embodiment) relating to the present invention.
In this embodiment, in the water reservoir tank 12, a water-current conveying mechanism 13 is provided as the second conveying means. This water-current conveying mechanism 13 includes a water inlet 13a and a water outlet 13b both provided at lower portions of the water reservoir tank 12. In operation, as water is introduced through the water inlet 13a and discharged through the water outlet 13b, there is generated a water current inside the water reservoir tank 12 which current can convey or carry the objects M heat-pasteurized and dropped into the water reservoir tank 12 one after another in a continuous manner to the outside of the enclosure member 3.
That is, the water reservoir tank 12 in this embodiment provides the three functions of the steam rise restricting mechanism, the cooling unit and the second conveying means. As a result, the construction of the pasteurizing device 1 can be even more simplified.

### [Fifth Embodiment]

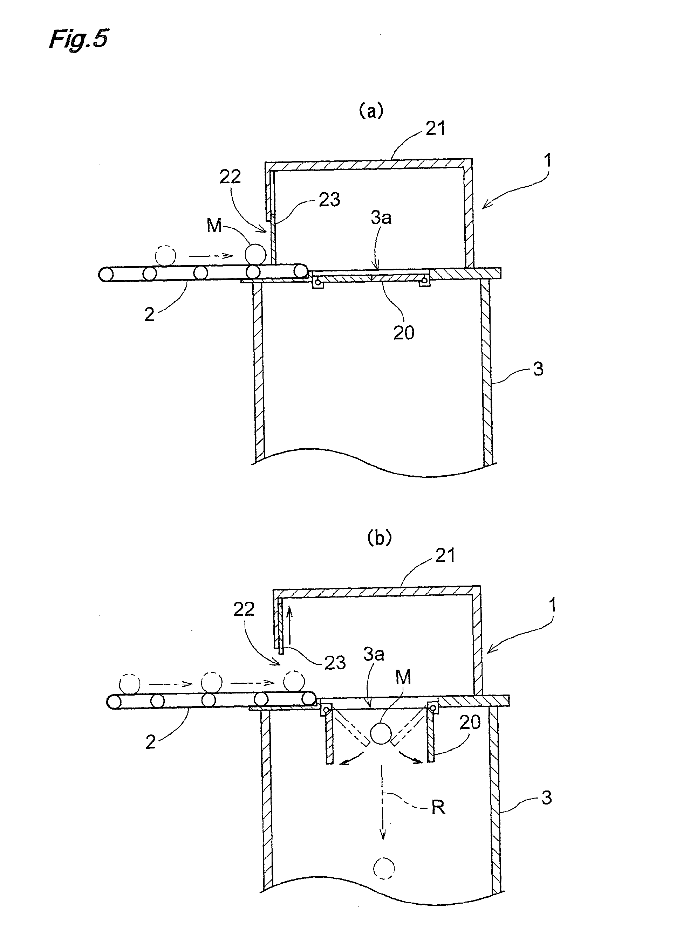

In the Fifth Embodiment, as shown in Fig. 5, at the upper opening portion 3a as the charging portion for the treated objects M, there is mounted an opening/closing lid 20 to be vertically pivotable to be freely opened and closed. That is, this opening/closing lid 20 constitutes the steam rise restricting mechanism.
Therefore, before the treated object M which has been conveyed by the first conveyer 2 is charged and dropped through the charging portion, the opening/closing lid 20 is opened (Fig. 5 (b)). Then, upon completion of the charging, the opening/closing lid 20 is closed at once in order to restrict rising escape of the steam present inside the dropping passageway R (Fig. 5(a)).
Incidentally, upwardly of the upper opening portion 3a, there is provided a dropping box 21 which surrounds the charging portion and at an entrance 22 provided at one side portion of this dropping box 21 for entrance/exit of the treated object from the first conveyer 2, there is provided a shutter 23 which is openable and closable. These arrangements altogether make the escape of steam even more difficult.

### [Sixth Embodiment]

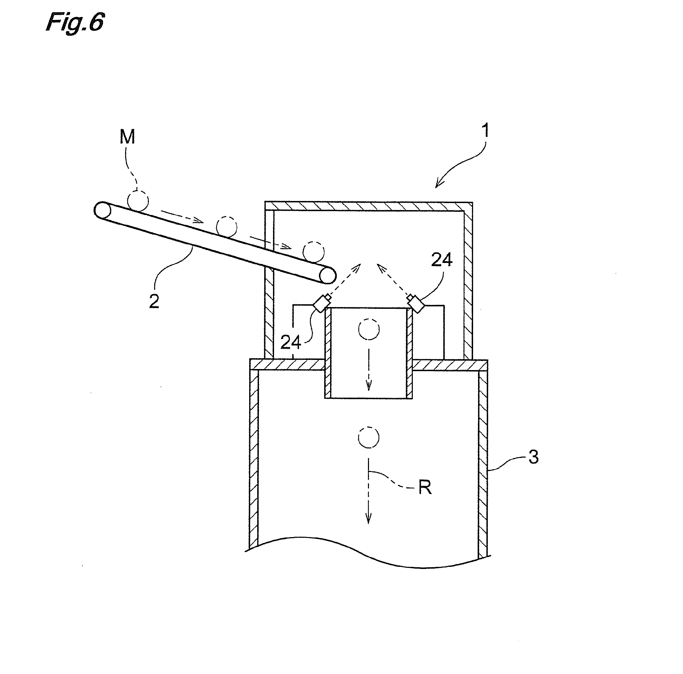

Fig. 6 shows the Sixth Embodiment, in which the steam rise restricting mechanism is constituted by air curtains 24 provided at the two opposed sides of the upper opening portion 3a.
This arrangement has the advantage of making introduction (charging) of the treated objects easier than the mechanically opened/closed opening/closing lid 20 described above.

### [Other Embodiments]

[1] The enclosure member 3 in the foregoing embodiments can be configured to be vertically expandable and contractible, so as to allow free adjustment of the length of the dropping passageway R.
[2] In the foregoing embodiments, instead of saturated steam, steam (70°C to 95°C) containing an appropriate amount of superheated steam (100°C to 170°C), or noncondensable gas (e.g. air, etc.) may be employed.
[3] The treated objects M in the foregoing embodiments are not limited to fresh food products, but can be processed food products or non-food products of resin or metal, or can even be plants which are used as raw material for Chinese harbal medicine or spice.
[4] In the Third through Sixth Embodiments described above, the baffle plates 11 provided in the Second Embodiment may be used and set appropriately.
[5] To the steam supplied to the dropping passageway R, as a substance providing pasteurizing effect or bacteria propagation restricting effect, e.g. acetic acid, ethanol, allyl isothiocyanate, vegetal oil, etc. may be added to come into contact with the surface of the treated object together with the steam inside the dropping passageway R. With this, even higher pasteurizing effect can be achieved. Results of experiments are shown in Table 8 given below.
This Table 8 shows the results of modes of treatment (non-treated, treated with steam alone, the treatment with steam with addition of restricting substance thereto), and the respective results thereof (the number of common bacteria. In this table, the language "hygrothermal pasteurization treatment" means the pasteurizing treatment described so far including the step of dropping objects into steam zone.

**[Table 8]**

| treated object | mode of treatment | number of common bacteria (cfu/g) |
|---|---|---|
| A: garland chrysanthemum | non-treated | 3.92E +06 |
| | hygrothermal pasteurization treatment | 2.51E+06 |
| | hygrothermal pasteurization + acetic acid treatment | 1.24E+05 |
| D: Chinese Cabbage | non-treated | 1.24E+06 |
| | hygrothermal pasteurization treatment | 8.85E+04 |
| | hygrothermal pasteurization + acetic acid treatment | 4.27E+04 |

Incidentally, the additive amounts of the above substance were 0.2 to 30%, preferably 0.5 to 2.0% in mass relative to steam supplied.
In the experiments in Table 8, acetic acid was added by 0.8 mass%.
If the amount is lower than the lower limit indicated above, it becomes difficult to obtain the pasteurizing effect or bacterial propagation restricting effect of the addition of the substance. Also, if the amount is increased over the upper limit value indicated above, there occurs no drastic increase in the effect of the addition.

### Industrial Applicability

The pasteurizing method and pasteurizing apparatus of the present invention is effective in particular for pasteurization of the outer surface of a treated object such as a fresh food product of vegetable, fruit or the like, in a short time without changing the quality of its inner part.

## Claims

1. A method of pasteurizing an object to be treated by heating the object with steam, comprising the steps of dropping the object into a steam zone and causing the dropping object to contact the steam.

2. The pasteurizing method according to claim 1,
wherein said steam is saturated steam.

3. The pasteurizing method according to claim 1 or 2,
wherein said treated object is a fresh food product.

4. The pasteurizing method according to any one of claims 1-3, wherein a substance providing a pasteurizing effect or an effect of restricting propagation of bacteria is added to the steam.

5. An apparatus for pasteurizing an object to be treated by heating the object with steam, the apparatus comprising:
a treated object charging portion provided at an upper end of the apparatus;
a treated object discharging portion provided at a lower end of the apparatus;
said treated object charging portion and said treated object discharging portion being communicated to each other;
an enclosure member defining therein a dropping passageway for causing the treated object to drop from said charging portion to said discharging portion;
a first conveying means for conveying the treated object to said charging portion;
a steam supplying unit for supplying steam to the dropping passageway of the enclosure member;
a steam rise restricting mechanism for restricting rising of steam supplied to the dropping passageway; and
a second conveying means for receiving the object dropped in the dropping passageway and conveying the object to the outside of the enclosure member;
wherein as said object is conveyed to the charging portion by the first conveying means and dropped, the object comes into contact with the steam supplied from the seam supplying unit in the dropping passageway, thus being pasteurized by heating.

6. The pasteurizing apparatus according to claim 5, wherein said steam rise restricting mechanism comprises an opening/closing lid provided at said charging portion, with the lid being openable/closable.

7. The pasteurizing apparatus according to claim 5, wherein said steam rise restricting mechanism comprises an air curtain provided at the charging portion.

8. The pasteurizing apparatus according to anyone of claims 5-7, wherein there is provided a cooling unit for cooling the object that has contacted the steam.

9. The pasteurizing apparatus according to any one of claims 5-8, wherein said steam supplying unit includes an adding means for adding to the steam a substance having a pasteurizing effect or an effect of restricting bacteria propagation.

## Amended claims

### Amended claims under Art. 19.1 PCT

**1.** A method of pasteurizing an object to be treated by heating the object with steam, comprising the steps of free falling the object in a perpendicular dropping passageway including a cooling unit supplied with cooling water and disposed perpendicularly below a steam zone supplied with saturated steam;
subsequently causing the entire surface of the object with the saturated steam in said steam zone to be pasteurized thereby; and
subsequently introducing the object free-fallen in the steam zone to said cooling unit to cause the entire surface of the object to contact the cooling water to be cooled thereby.

**2.** The pasteurizing method according to claim 1, wherein said treated object is a fresh food product.

**3.** The pasteurizing method according to claim 1, wherein a substance providing a pasteurizing effect or an effect of restricting propagation of bacteria is added to the steam.

**4.** An apparatus for pasteurizing an object to be treated by heating the object with steam, the apparatus comprising:
a treated object charging portion provided at an upper end of the apparatus;
a treated object discharging portion provided at a lower end of the apparatus;
said treated object charging portion and said treated object discharging portion being communicated to each other;
an enclosure member defining therein a free-fall dropping passageway for causing the treated object to drop from said charging portion to said discharging portion;
a first conveying means for conveying the treated object to said charging portion;
a steam supplying unit for supplying saturated steam to the free-fall dropping passageway of the enclosure member to form a steam zone;
a cooling unit for forming a cooling portion perpendicularly below the steam zone for receiving cooling water;
a steam rise restricting mechanism for restricting rising of steam supplied to the dropping passageway; and
a second conveying means for receiving the object dropped to the discharging portion and conveying the object to the outside of the enclosure member;
wherein as said object is conveyed to the charging portion by the first conveying means and dropped in the free-fall dropping passageway, the entire surface of the object comes into contact with the saturated steam in the steam zone, thus being pasteurized by heating; and
subsequently, the object having dropped in the free-fall dropping passageway is introduced to the cooling unit, so that the entire surface of the object comes into contact with the cooling water to be cooled thereby.

**5.** The pasteurizing apparatus according to claim 4, wherein said steam rise restricting mechanism comprises an opening/closing lid provided at said charging portion, with the lid being openable/closable.

**6.** The pasteurizing apparatus according to claim 4, wherein said steam rise restricting mechanism comprises an air curtain provided at the charging portion.

**7.** The pasteurizing apparatus according to any one of claims 4 to 6, wherein said steam supplying unit includes an adding means for adding to the steam a substance having a pasteurizing effect or an effect of restricting bacteria propagation.
